# EUROPEAN PATENT SPECIFICATION

(11) **EP 0 971 601 B1**
(45) Date of publication and mention of the grant of the patent: **21.06.2006**
(21) Application number: 98914888.7
(22) Date of filing: 16.03.1998
(51) Int. Cl.: A23L 1/30, A61K 36/00, A61Q 19/00, A23F 3/34

(54) **DECAFFEINATED MATE EXTRACTS AND THE USE THEREOF**
ENTKOFFEINIERTE MATE-EXTRAKTE UND DEREN VERWENDUNG
EXTRAIT DE MATE DECAFEINE ET UTILISATION

(30) Priority: 21.03.1997 IT MI970663
(43) Date of publication of application: 19.01.2000
(73) Proprietor: Sensient Flavors Italy S.R.L., 20099 Sesto San Giovanni (IT)
(72) Inventor: MAFFEI FACINO, Roberto, Uni. degli Studi di Milano, I-20131 Milano (IT); CARINI, Marina, Uni. degli Studi di Milano, I-20131 Milano o (IT); MARIANI, Marco, I-20099 Sesto San Giovanni (IT)
(74) Representative: Minoja, Fabrizio
(86) International application number: PCT/EP1998/001517
(87) International publication number: WO 1998/042209

(56) References cited:
- BR-A- 9 303 217
- CH-A- 626 781
- DE-A- 3 313 339
- US-A- 2 115 411
- PATENT ABSTRACTS OF JAPAN vol. 096, no. 007, 31 July 1996 & JP 08 070772 A (UNIE CAFE:KK), 19 March 1996
- A. VAZQUEZ: "Studies on mate drinking" JOURNAL OF ETNOPHARMACOLOGY, vol. 18, 1986, pages 267-272, XP002074703
- H. GRAHAM: "Maté" PROG. CLIN. BIOL. RES., no. 185, 1984, pages 179-183, XP002074704
- PATENT ABSTRACTS OF JAPAN vol. 018, no. 437 (C-1238), 16 August 1994 & JP 06 135848 A (TAIYO KAGAKU CO LTD), 17 May 1994
- A. GUGLIUCCI: "Low density lipoprotein oxidation is inhibited by extracts of ilex paraguariensis" BIOCHEMISTRY AND MOLECULAR BIOLOGY INTERNATIONAL, vol. 35, no. 1, 1995, pages 47-56, XP002074705 cited in the application
- A. CAMPOS: "The total reactive antioxidant potential and total antioxidant reactivity of Ilex paraguayensis extracts and red wine" J. BRAZ. CHEM. SOC., vol. 7, no. 1, 1996, pages 43-49, XP002074706

## Description

The present invention relates to decaffeinated Maté extracts, characterized in caffeic acid derivatives and standardized in polyphenol content, to their use as antioxidants and radical scavengers in the preparation of medicaments, dietary supplements, cosmetics, and to the pharmaceutical, cosmetic, alimentary/dietary compositions containing them.

It is well known from epidemiological and clinical studies [1] that the intake of antioxidants/radical scavengers through the diet or their administration through pharmaceutical and nutritional products is of great benefit in wellness, health maintenance, and in the prevention of chronic and degenerative diseases associated to oxidative damage (atherosclerosis, cancer, coronary heart disease, rheumatoid arthritis, Alzheimer's and Parkinson's diseases and aging), since a free radical overload is massively involved in their development [2-7]. In the cosmetic field, compounds with antiradicalar activity able to enhance the natural defenses of the skin, hold the greatest promise for the development of photoprotective agents, since free radicals generated on epidermal skin layer by ultraviolet exposure, are recognized as potent inducers of premature skin aging and skin cancer, in particular of malignant melanoma [8].

Maté tea is one of the most consumed stimulating beverages in several South American countries, including Uruguay, Paraguay, Argentina and Brazil, prepared as a hot infusion of the caffeine-rich leaves of *Ilex Paraguayensis* St. Hill (Aquifoliaceae) and of other Ilex species. As occurs for other widely used stimulating beverages like coffee and tea, its use is linked to the presence of caffeine, responsible for the stimulating effects on central nervous system and the heart.

While the use of Maté extracts in cosmetic or pharmaceutical products since the presence of xanthines is known [9,10], the use of decaffeinated extracts from Maté as natural antioxidants/radical scavengers is not documented.

JP08070772 (Patent Abstracts of Japan, vol. 096, no. 007, 31 July 1996) discloses the preparation of caffeine-reduced mate tea extracts.

Vazquez, A. et al. (Journal of Etnopharmacology, vol. 18, 1986, pages 267-272) discloses an extract from plants of Ilex Paraguayensis and a method of preparation that reduces the caffeine content.

DE 3313339 discloses mate decaffeinated extracts and a process for the preparation thereof.

Although in two publications [11,12] has been reported the antioxidant action of water infusions of Mate! leaves on the Cu²⁺-induced peroxidation of isolated low density lipoproteins, and in other two patents Mate extracts have been proposed as antioxidant/radical scavengers [13,14], surprisingly it has been found that the decaffeinated extracts of the invention, enriched in well characterized polyphenols and standardized in polyphenols/chlorogenic acid content, possess a significantly greater radical scavenging activity against all free radicals involved in the onset (superoxide anion O₂⁻, hydroxyl radical HO°; preventive antioxidants) and the propagation (peroxyl radical LOO°; chain-breaking antioxidants) of the peroxidation processes.

According to the invention, decaffeinated extracts from plants of Ilex species are used in the preparation of pharmaceutical, cosmetic or dietary/alimentary compositions.

Preferably, the extracts of the invention are obtained from plants of Ilex Paraguayensis species, and they contain no more than 0,1% by weight of caffeine.

Such extracts have been prepared according to the following procedure:
Leaves of *Ilex Paraguayensis* dried and finely minced were mixed with boiling water (50 mg/ml) under mechanical stirring and cooled; the extracts were centrifuged at 10,000 x g for 10 min and the supernatant exhaustively extracted with chlorinated solvents, preferably methylene chloride, to eliminate caffeine, theobromine and other not phenolic substances.

The aqueous residue were concentrated in vacuo to dryness to obtain a pale-yellow powder (yield = 23%), the decaffeinated aqueous extract of Maté.

The total or partial decaffeination process of Maté, in analogy to coffee or tea, could be done by several other techniques, as liquid-solid extraction, liquid-liquid extraction, chromatographic separation, supercritical or subcritical extraction (CO₂, etc.), and so on.

Alternatively decaffeination could be carried out directly on the Maté leaves before the extraction of the final polyphenol-rich extract.

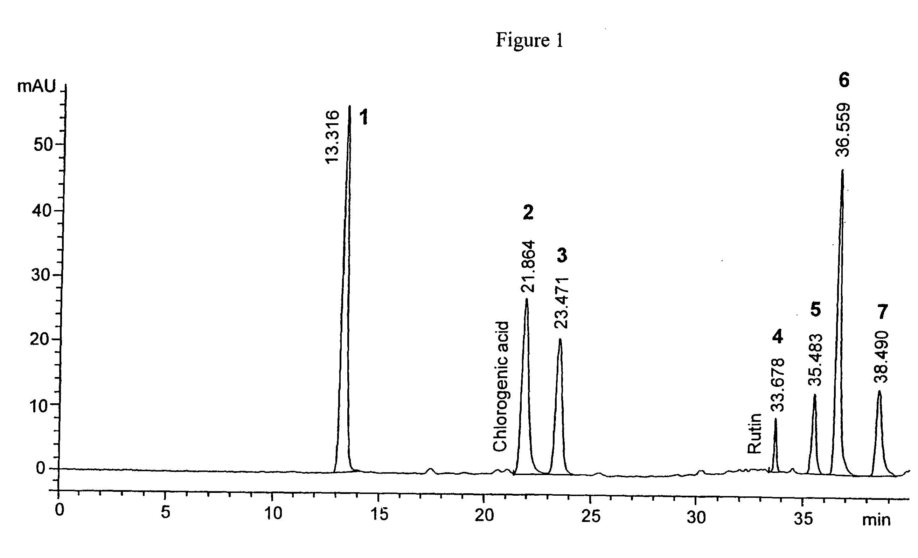

The HPLC profile of the decaffeinated aqueous extract of Mate (Fig.1) obtained according to the above process, indicates the presence of at least 7 peaks with absorption maximum at 330 nm, typical of chlorogenic, caffeoyl derivatives [15] and flavonoids: all the components of the polyphenol fraction were characterized by LC-MS analysis.

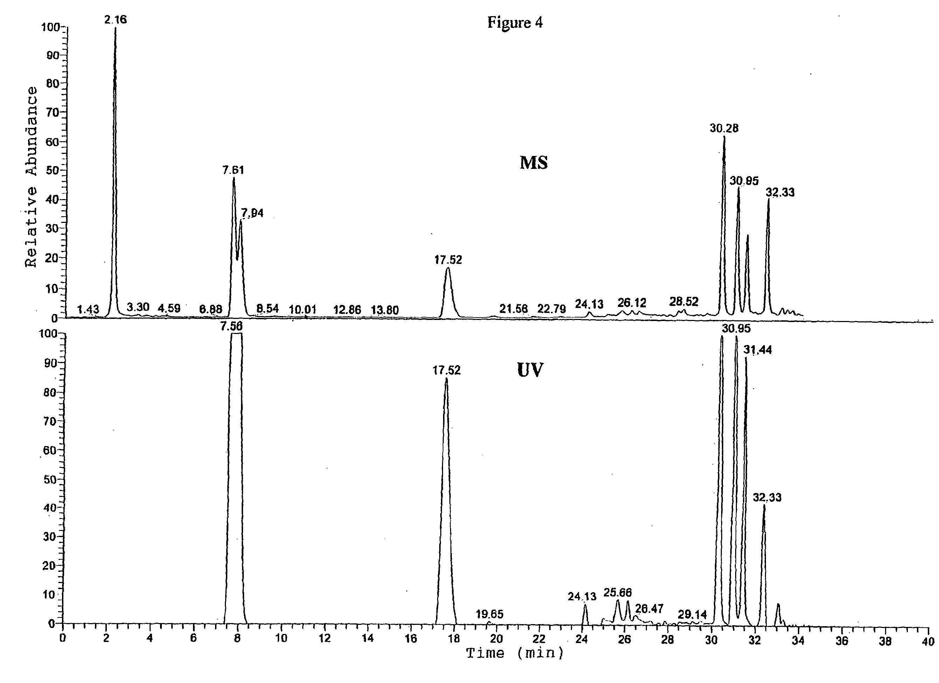

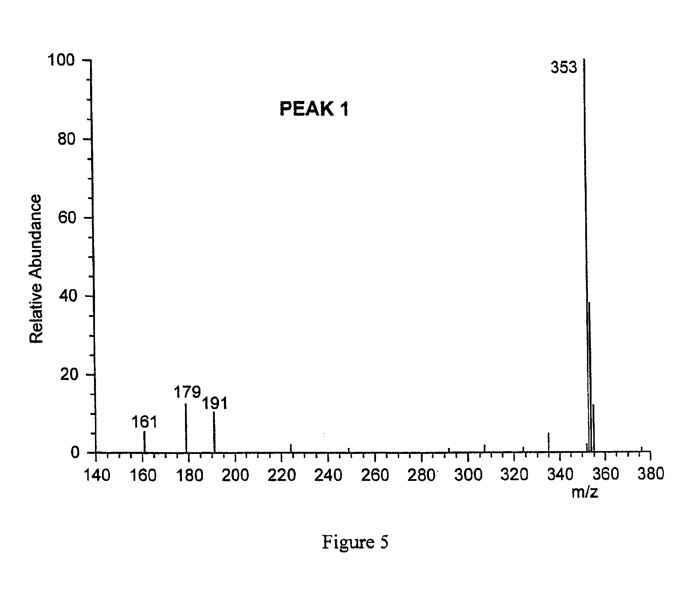

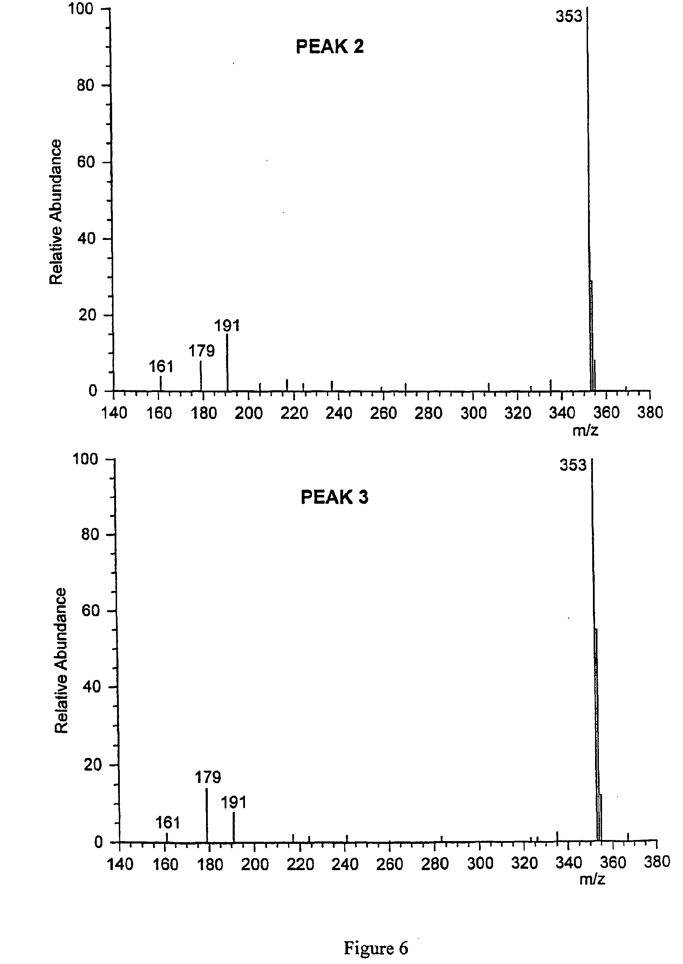

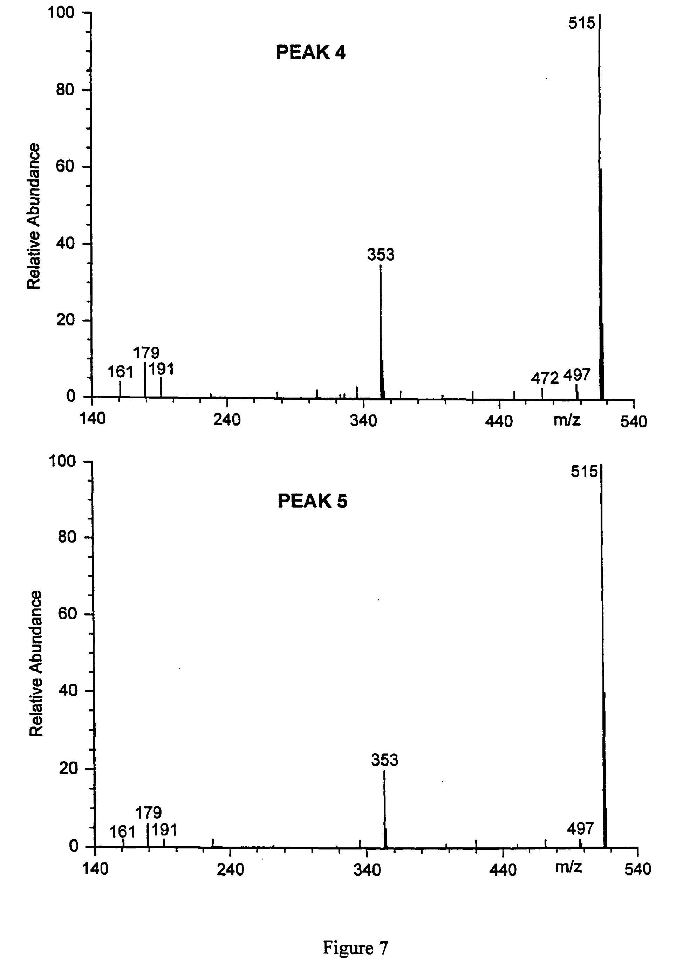

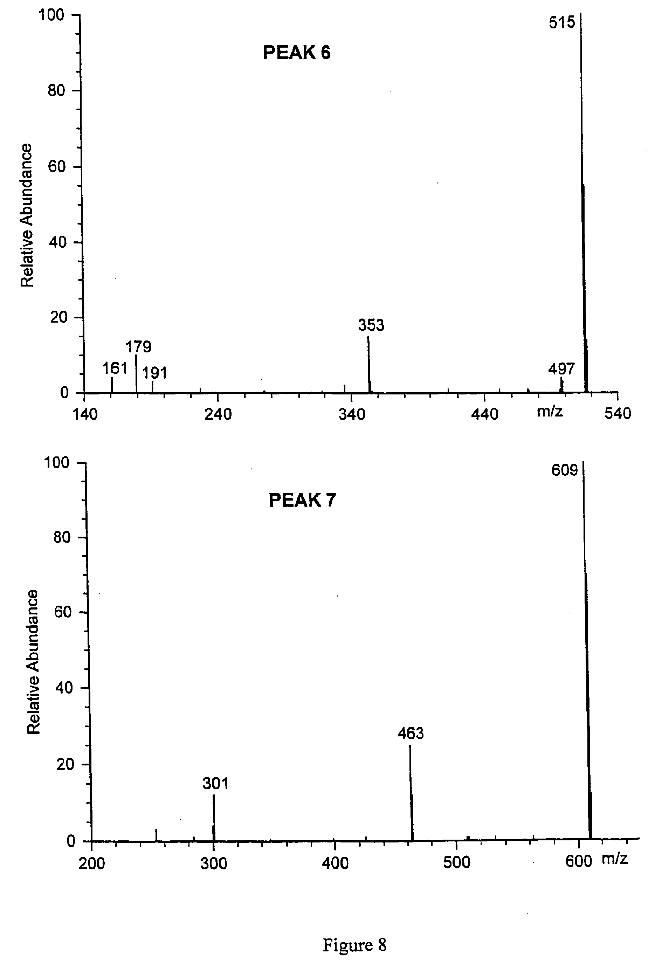

In Fig. 4 is shown a typical mass chromatogram (in the bottom box the corresponding UV profile at 330 nm), in which 7 main peaks are detectable, and in Fig. 5-8 the negative ion mass spectra of the single components are shown. Peaks 1-3 show a molecular ion [M-H]⁻ at m/z 353 (M.W. 354) and fragmentation patterns with ions at m/z 179 [Caffeic acid-H]⁻ and m/z 191 [quinic acid - H]⁻ consistent with the structures of caffeoylquinic acid derivatives [16], peak 2 being chlorogenic acid (3-O-caffeoyl quinic acid), as determined by comparison with the authentic standard. Peaks 1 and 3 were identified as isomers of chlorogenic acid (i.e. isochlorogenic acid = 5-O-caffeoyl quinic or neochlorogenic acid, pseudochlorogenic acid), but in the absence of standards, not commercially available, no definitive structure assignement can be done for peaks 1 and 3. Peaks 4-6 show molecular anion at m/z 515 (M.W. 516), and similar fragmentation pattern, with the diagnostic peak at m/z 353 due to loss of a caffeoyl residue (162 µ) [M - Caff - H]- and the ions at m/z 179 and 191, tipical of dicaffeoyl esters of quinic acid [16]. On the basis of molecular weight and the fragmentation patterns we have detected and identified in this polyphenol fraction three isomeric dicaffeoyl esters (i.e. 3,4-O-dicaffeoyl, 4,5-O-dicaffeoyl, 3,5-O-dicaffeoyl or 1,5-dicaffeoyl esters of quinic acid). By contrast, peak 7 shows a molecular anion [M-H]⁻ at m/z 609 (M.W. 610) and a fragmentation pattern typical of glycosyl derivatives (losses of terminal sugars) with ions at m/z 463 [M-H-Rha]⁻ and m/z 301 [M-H-Rha-Glu]⁻, this last corresponding to the deprotonated molecular ion of quercetin. On the basis of the fragmentation pattern and by comparison with the LC-MS profile of the reference standard, to peak 7 was assigned the structure of rutin (quercetin-3-rutinoside).

The total polyphenol content of the decaffeinated aqueous extract of Mate obtained according to the described procedure was 50 ± 0.8 % (w/w), determined by the Folin-Ciocalteau method [17]; the content in chlorogenic acid, determined by HPLC analysis (Fig. 1a, peak 2, R.T. 21.86 min) was 5.1 ± 0.2% and assuming the same extinction coefficient for chlorogenic acid isomers, the total content of chlorogenic acids was 17.7 ± 0.5 % (8.2 % peak 1 Fig.1a, R.T. 13.3; 4.4 % peak 3 Fig.1a, R.T. 23.4); the content in rutin (peak 4, R.T. 33.68) was 1.96 ± 0.3 %.

In most cases the caffeine content of the preparations was below 0.1%.

The extracts of the invention have been examined for their antilipoperoxidant and radical scavenging activities in different experimental models like cell-free, membranous and cellular systems. The antilipoperoxidant (chain breaking) activity resulted much greater than that of a conventional Maté infusion, both in terms of IC₅₀ value (8.1 vs 12.5 µg/ml), and of concentration which gives total inhibition of the peroxidation process (20 vs 37.5 µg/ml) [11,12]. The overall antioxidant activity of the decaffeinated extracts, determined as quenching ability of the stable radical DPPH (see below), resulted to be even greater than that of a conventional Maté infusion (IC₅₀ = 4 µg/ml vs 6.2 µg/ml). The radical scavenging activity of the extracts of the invention is due to the presence of low molecular weight caffeic acid derivatives, chlorogenic acid and other congeners, which have been fully characterized by means of HPLC and LC-MS. However, the tests have demonstrated that chlorogenic acid is less active than the Mate extract as radical scavenging agent, indicating a cooperative antioxidant interaction between the polyphenol components of the decaffeinated Maté extract (see table 1).

The decaffeinated extracts of Maté can be used alone or in combination with other natural extracts and/or different substances, such as hydrophilic/lipophilic antioxidants, natural/synthetic flavors in foods, beverages, dietary, cosmetic and pharmaceutical products; in addition they can be formulated with suitable vehicles, excipients or additives, according to the techniques conventionally used in the pharmaceutical, cosmetic and alimentary fields.

The decaffeinated extracts standardized in their polyphenol contents can be both in liquid, liquid viscous, or solid (powder, granules, etc.) physical forms. They can also be in a powder form obtained by a spray drying process on suitable supporting agents like sugars, maltodextrines, gums, etc.

A further object of the present invention relates to pharmaceutical, cosmetic, alimentary/dietary compositions containing the extracts of the invention, which are, in pharmaceutical forms such as tablets, capsules, sachets, syrups, suppositories, vials and ointments, in cosmetic forms such as emulsions, gels, lotions or powders and dietary forms such as foods, beverages and dietary products, are specifically suited as antioxidants and radical scavengers, in the prevention/treatment of free radical-mediated pathologies, such as atherosclerosis, cancer, cardiovascular diseases, rheumatoid arthritis, neurological disorders (Alzheimer's and Parkinson's diseases), aged-related cataract, infective diseases and in the maintenance of the immune function, as photoprotectants to prevent the accelerated aging of the skin and the phootoxidative degeneration of the skin and the hair, as dietary supplements for the prevention of the physical aging, and for the maintenance of the physical performance under strenuous exercise, for professional athlets.

In particular, in the prevention/tretament of all the free-radical mediated physiopathological situations, the product of the invention can be advantageously combined with other endogenous antioxidants, such as carotenoids, vitamin E, ascorbic acid, glutathione and sulphurated aminoacids, in order to preserve and maintain the integrity of the physiological antioxidant pool through a maximization of the antioxidant effect due to synergistic interaction.

### Brief description of the Figures

Fig.1 - HPLC profile of the decaffeinated aqueous extract of Maté. Chromatographic conditions: reverse phase column LC-18; mobile phase: linear gradient of 100% A (0.0127 N H₃PO₄/CH₃CN/Ethylacetate 96.5'/3/0.5) to 30% B (CH₃CN) in 30 min; flow rate: 1 ml/min; detection: UV-DAD 330 nm. Seven peaks are detectable with absorbption maximum at 330 nm, typical of chlorogenic acid, caffeoyl derivatives [15] and flavonoids.

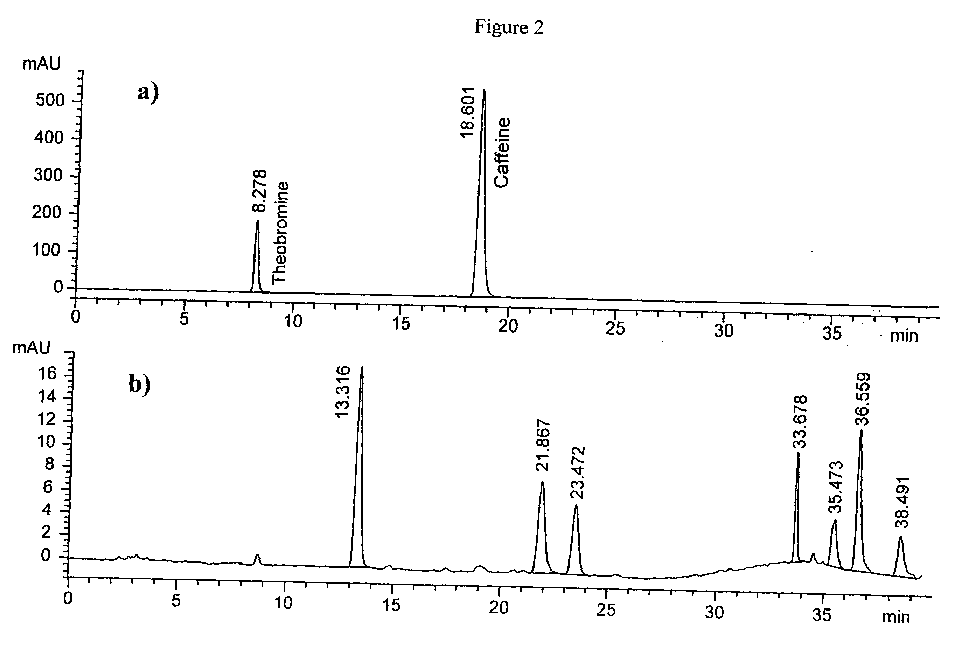

Fig. 2a - HPLC profile (detection 270 nm) of the methylene chloride extract, containing caffeine (R.T. 18.63 min) and theobromine (R.T. 8.28 min).
Fig.2b - HPLC profile (detection 270 nm) of the decaffeinated aqueous extract of Matè (see the absence of caffeine at 18.63 min and of theobromine at 8.28 min).
Fig.3 - HPLC chromatograms (detection 330 nm) of the decaffeinated aqueous extract of Matè before (a) and after reaction with DPPH (b = 0.0625 mM; c = 0.125 nM; d = 0.25 mM; e = 0.5 mM).
Fig.4 - LC-MS profile of the decaffeinated aqueous extract of Mate (bottom box UV tracing). Experimental conditions: Finnigan LCQ mass spectrometer; reverse phase column LC-18; mobile phase: linear gradient of 100% A (95% ammonium formiate / 5% CH₃CN, pH 4.5 with HCOOH) to 30% B (95% CH₃CN / 5% ammonium formiate, pH 4.5 with HCOOH) in 30 min; flow rate: 1 ml/min; negative ion detection.
   Peak 1 = [M-H]⁻ m/z 353 chlorogenic acid isomer; peak 2 = [M-H]- m/z 353 chlorogenic acid; peak 3 = [M-H]⁻ m/z 353 chlorogenic acid isomer; peaks 4, 5, 6 = [M-H]- m/z 515 dicaffeoyl esters of quinic acid; peak 7 = [M-H]- m/z 609 rutin.
Fig.5 - Negative ion ElectroSpray (ES) mass spectrum of peak 1.
Fig.6 - Negative ion ElectroSpray (ES) mass spectra of peaks 2 and 3.
Fig.7 - Negative ion ES mass spectra of peaks 4 and 5.
Fig.8 - Negative ion ES spectra of peaks 6 and 7
Fig.9 - Time-course of conjugated dienes in the propagation and breakdown phases of lipid peroxidation in phosphatidylcholine liposomes: chain-breaking antioxidant activity of decaffeinated aqueous extract of Maté. Values are the means ± S.D. of 5 determinations.
Fig.10 - Protective effect of decaffeinated aqueous extract of Maté on UVB-induced hemolysis. Red blood cells (0.2% suspensions) were exposed to UVB radiation for 210 min in the absence and in presence of different concentrations of decaffeinated aqueous extract of Maté; percentage hemolysis was turbidimetrically determined at 710 nm at 30 min intervals. Values are the means ± S.D. of 5 determinations.

The following examples illustrate the invention in more details.

### Example 1

### Antioxidant and radical scavenging activity assays of decaffeinated Matè extracts

### a) 2,2-Dyphenyl-1-picrylhydrazyl (DPPH) radical

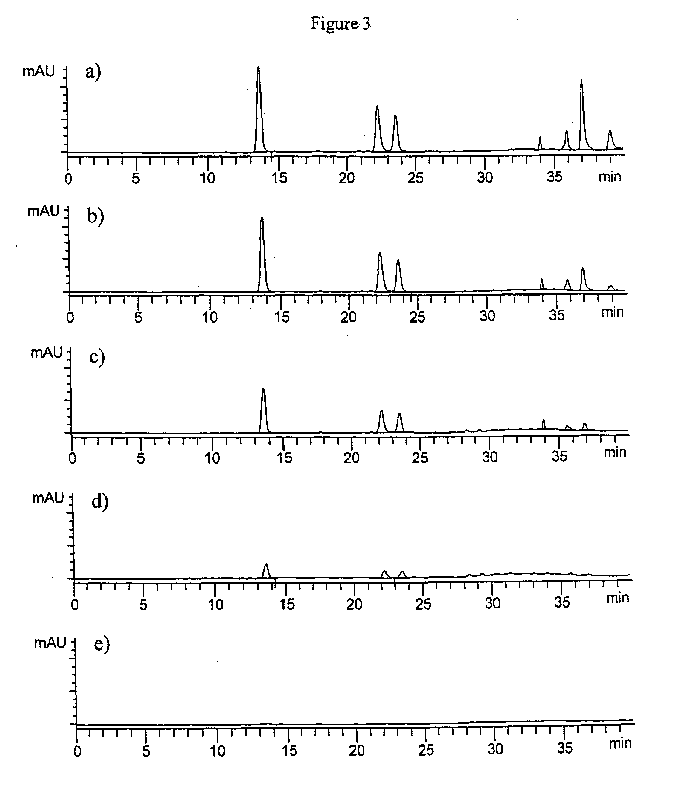

The activity of the extract in quenching the stable free radical DPPH through hydrogen transferring reaction, has been tested by monitoring the loss in absorbance at 517 nm of a methanol solution of DPPH [18] in the presence and the absence of increasing concentrations of Maté extract. The results, summarized in Table I, indicate that Matè extract (IC₅₀ = 4.2 pg/ml), although equipotent to chlorogenic acid (IC₅₀ = 4.34), is significantly more effective than ascorbic acid or vitamin E (IC₅₀ = 19 µg/ml) in quenching DPPH radical. All the components detected by HPLC analysis (Fig.1), contribute to the DPPH quenching ability of the extract, since after reaction with increasing concentrations of f DPPH (0.625, 0.125, 0.25, 0.5 mM), a progressive disappearance of peaks 1-7 is observed (Fig.3).

### b) Superoxide anion

The decaffeinated aqueous extract of Maté dose-dependently inhibit the rate of reduction of nitrotetrazolium blue chloride (NBT) by superoxide anion generated in the phenazine methosulfate/NADH system [18], with a minimal effective concentration of 5 µg/ml and an IC₅₀ value of 15 µg/ml; chlorogenic acid was less potent in entrapping superoxide radicals, with an IC₅₀ value of 24 µg/ml (Table I). The bimolecular rate constant for the reaction of superoxide radicals with Maté extract, determined by kinetic competition studies [18] gives a *K* value of 1.2 x 10⁵ M⁻¹ cm⁻¹, more favourable than that of chlorogenic acid (K = 0.5 x 10⁵ M⁻¹ cm⁻¹).

### c) Hydroxyl radical

Using the deoxyribose assay [19], it has been demonstrated that the decaffeinated aqueous Mate extract inhibit the HO°-induced deoxyribose degradation, as indicated by the progressive decrease in thiobarbituric acid-malondialdehyde (TBA-MDA) chromogen formation in the presence of increasing concentration of the extract. The rate constant of reaction of Maté extract with HO° (*K*), calculated according to Aruoma [19] using a *K* value for deoxyribose of 3.1 x 10⁹ M⁻¹ s⁻¹, was 4.5 ± 0.3 x 10⁹ M⁻¹ s⁻¹. Chlorogenic acid was less effective than Maté extract, with a *K* value of 2.1 ± 0.2 x 10⁹ M⁻¹ s⁻¹ (Table I).

### d) Antilipoperoxidant activity in phosphatidylcholine liposomes

The chain-breaking antioxidant activity of the decaffeinated aqueous extract of Matè was determined in a well established model of HO°-induced peroxidation (by ultrasounds exposure) of phosphatidylcholine liposomes, by monitoring the formation of conjugated hydroperoxides during the propagatory phase of the peroxidation process [20-22]. Maté extract markedly and dose-dependently inhibited the increase in conjugated dienes (6 hours after induction), by 17% at 1 µg/ml, 36% at 5 µg/ml, 66% at 10 µg/ml and complete inhibition was observed at 20 µg/ml (IC₅₀ = 8.1 µg/ml); chlorogenic acid was less effective as chain-breacking antioxidant, with an IC₅₀ value of 18.4 µg/ml (Table I).

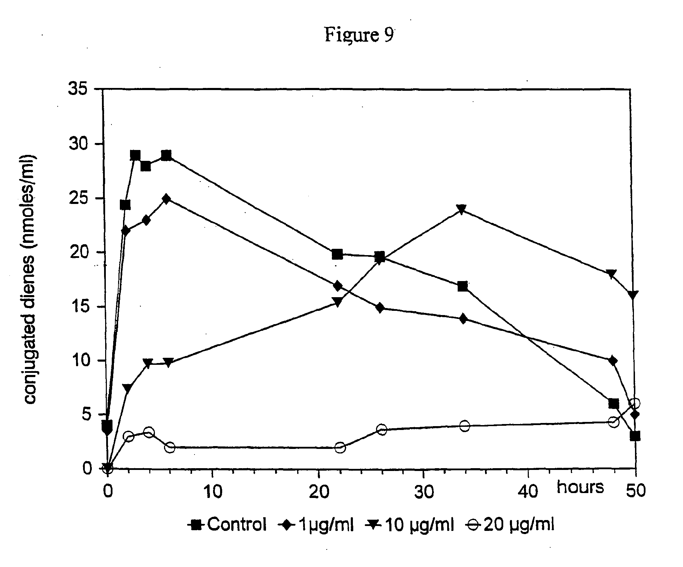

In addition, as shown in Fig. 9, in the samples protected with Maté extract, the plateau of conjugated dienes was reached later than the control (t = 6h), with a lag time of 28 hours at 10 µg/ml (t = 34 h); at the highest concentration the conjugated dienes were far from the plateau even after 50 h.

### e) UVB-induced lysis of rat erythrocytes (RBC)

Finally we evaluated in a cellular model (0.2% erythrocyte suspensions) the protective effect of the decaffeinated aqueous extract of Matè on the peroxidative stress induced by UVB exposure (0.4-4 J/cm²) [23], and monitoring the time-course of hemolysis (turbidimetric determination at 710 nm) at 30 min intervals. The protective effect of Maté extract (1-20 µg/ml) on UVB-induced hemolysis was compared to that of chlorogenic acid (1-20 µg/ml).

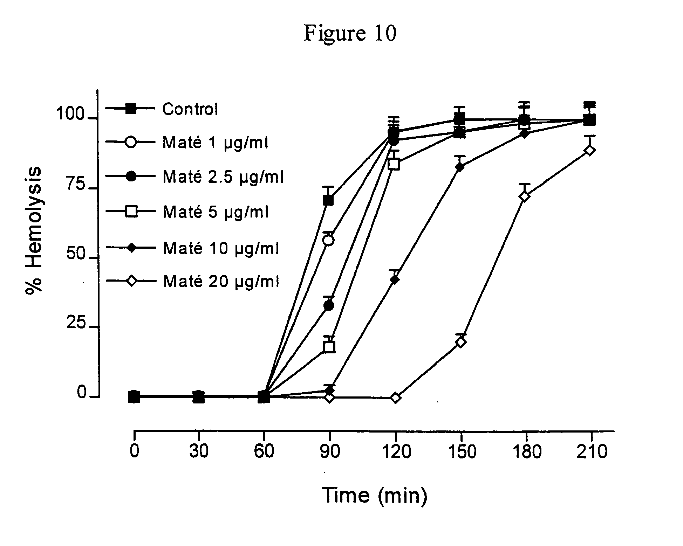

As shown in Fig. 10, hemolysis in the controls starts after 60 min irradiation and plateaued between 90 and 120 min (71.2 ± 4.8 % and 95.5 ± 5.2% hemolysis).

The decaffeinated aqueous extract of Maté significantly and dose-dependently delay the onset of the UVB-induced hemolysis, by 30 min at 10 µg/ml and by 60 min at 20 pg/ml; the protective effect starts from low concentrations, since at 1 µg/ml % hemolysis at 90 min irradiation was only 56.6 ± 2.8 %, 33.0 ± 3.2 % at 2.5 µg/ml and 18.2 ± 2.6 % at 5 µg/ml (IC₅₀ determined at 90 min = 2.35 µg/ml). Chlorogenic acid at the same concentrations (data not shown) was slightly less effective than decaffeinated aqueous extract of Maté, with an IC₅₀ value (90 min irradiation) of 3.2 µg/ml.

**Table I - Radical scavenging activity of decaffeinated aqueous extract of Maté**

| **EXPERIMENTAL MODEL** | **Maté extract** | **Chlorogenic acid** |
|---|---|---|
| DDPH radical | IC₅₀ (µg/ml) = 4.2 | IC₅₀ (µg/ml) = 4.3 |
| Superoxide anion (NBT reduction) | IC₅₀ (µg/ml) = 15.0 | IC₅₀ (µg/ml) = 24.2 |
| | *K* = 1.2 x 10⁵ M⁻¹s⁻¹ | K = 0.5 x 10⁵ M⁻¹s⁻¹ |
| Hydroxyl radical (deoxyribose assay) | K = 4.5 x 10⁹ M⁻¹ s⁻¹ | K = 2.1 x 10⁹ M⁻¹ s⁻¹ |
| Peroxyl radical (chain-brealdng antioxidant) | IC₅₀ (µg/ml) = 8.1 | IC₅₀ (µg/ml) = 18.4 |

### Example 2

1000 ml of diet beverage contain:
- Acesulfame K 0.09 g
- Aspartame 0.09 g
- Cyclamate 0.20 g
- Decaffeinated aqueous extract of Maté 1.00 g
- White grape juice 4 fold 5.00 g
- Citric acid 1.80 g
- Apple Flavor 1.00 g
- Citron flavor 0.20 g
- Water q.s

### REFERENCES

1. Frei B. *Natural Antioxidants in Human Health and Disease.* Academic Press, Inc., San Diego (CA) 1994.
2. Cross C.E. Oxygen radicals and human disease. *Ann. Intern. Med.,* 107, 526-45 (1987).
3. Ames B.N., Shigenaga M.K., Hagen T.M. Oxidants, antioxidants and the degenerative diseases of aging. *Proc. Natl. Acad. Sci.,* 90, 7915-22 (1993).
4. Halliwell B., Gutteridge J.M.C. eds. *Free Radicals in Biology and Medicine,* 2nd edition, Clarendon Press, Oxford (1989).
5. Sies H. ed. *Oxidative stress: Oxidants and Antioxidants.* Academic Press, London (1991).
6. Packer L. Oxidative stress and the antioxidant vitamins C and E in functional medicine. In *"Oxidative Processes and Antioxidants"* (Paoletti R., Samuelsson B., Catapano A., Poli A., Rinetti M. eds.) Raven Press, New York, 1994, pp. 153-64.
7. Cadenas E. and Packer L. eds. *Handbook of antioxidants,* Marcel Dekker Inc. (1996).
8. Fuchs J. and Packer L. Photooxidative stress in the skin. In *"Oxidative stress Oxidants and Antioxidants.* Academic Press, London (1991), pp 559-83.
9. Honerlagen H.J. and Bolardt S.M. Masse, welche insbesondere für die Einkapselung in ein festes Umhüllungsmaterial geeignet ist, und Verfahren zur Herstellung dieser Masse. EP 0 496 705 A1 (1992).
10. Voβ H., Deppe M., Deppe R. Kosmetisches Mittel mit anticellulitischer Wirkung. DE 44 01 308 A1 (1995).
11. Gugliucci A. and Stahl A.J.C. Low density lipoprotein oxidation is inhibited by extracts of *Ilex Paraguariensis, Biochem. Molec. Biol. Int.,* 35, 47-56 (1995).
12. Gugliucci A. Antioxidant effects of Ilex paraguayensis: induction of decreased oxidability of human LDL in vivo. *Biochem. Biophys. Res. Commun.,* 224, 338-44 (1996).
13. Kurose S., Matsumura T. Extracting flavonoids and catechins from Mate tea leaves for use as antioxidants and active agents in cosmetics. Braz.Pedido, PI BR 93 03,217.
14. Hibino M., Nakamura I., Sakanaka S. Extraction of oxygen radical scavengers from Ilex paraguayensis. Jpn. Kokai Tokkyo Koho JP 06,135,848 [94,135,848].
15. Clifford M.N. and Ramirez-Martinez J.R. Chlorogenic acids and purine alkaloids contents of Maté (Ilex Paraguayensis) leaf and beverage. *Food Chem.,* 35, 13-21 (1990).
16. Maffei Facino R., Carini M., Aldini G., Marinello C. Direct characterization of caffeoyl esters with antihyaluronidase activity in crude extracts from *Echinacea angustifolia* roots by fast Atom Bombardment Mass Spectromatry. Il Farmaco, 48, 1447-61 (1993).
17. Singleton S.L., Ross J.A. Colorimetry of total phenolics with phosphomolybdic-phosphotungstic acid reagents. *Am.J. Enol. Vitic.,* 16, 144-58 (1965).
18. Maffei Facino R., Carini M., Saibene L. Scavenging of free radicals by tenoxicam: a participating mechanism in the antirheumatic/antiinflammatory efficacy of the drug. *Arch. Pharm. Pharm. Med. Chem.,* 329, 457-63 (1996).
19. Aruoma I.O. Deoxyribose assay for detecting hydroxyl radicals. *Meth. Enzymol.,* 233, 57-66 (1994).
20. Maffei Facino R., Carini M., Aldini G., Bombardelli E., Morazzoni P., Morelli R. Free radical scavenging and anti-enzyme activities of procyanidines from *Vitis vinifera.* A mechanism for their capillary protective action. *Arzneim. Forsch.*/*Drug Res.,* 44, 592-601 (1994).
21. Maffei Facino R., Carini M., Aldini G., Saibene L., Morelli R. Differential inhibition of superoxide, hydroxyl and peroxyl radicals by nimesulide and its main metabolite 4-hydroxynimesulide. *Arzneim. Forsch.*/*Drug Res.,* 45, 1102-9 (1995).
22. Maffei Facino R., Carini M., Aldini G., Berti F., Rossoni G., Bombardelli E., Morazzoni P. Procyanidines from *Vitis vinifera* seeds protect rabbit hearth from ischemia/reperfusion injury: antioxidant intervention and/or iron/copper sequestering ability. *Planta Med.,* 62, 495-502 (1996).
23. Maffei Facino R., Carini M., Aldini G., Calloni M.T., Bombardelli E., Morazzoni, P. Sparing effect of procyanidins from Vitis vinifera L. on vitamin E: in vitro studies. *Planta Med.,* 64 (1998 In press).

## Claims

1. A decaffeinated aqueous extract from plants of *Ilex Paraguayensis* having the HPLC profile reported in Fig. 1 and containing no more than 0.1 % by weight of caffeine.

2. The extract of claim 1, containing 50 ± 0.8 % (w/w) polyphenols, as determined by the Folin-Ciocalteau method, 5.1 f 0.2 % (w/w) chlorogenic acid, as determined by HPLC analysis, 1.96 t 0.3 % (w/w) rutin.

3. The extract according to claims 1 or 2, for use as medicaments.

4. The extract of claim 3, for the prevention or treatment of pathologies associated with oxidative damage.

5. The extract of claim 4, for the prevention or treatment of atherosclerosis, cancer, cardiovascular diseases, rheumatoid arthritis, Alzheimer's disease, Parkinson's disease and other age-related chronic degenerative pathologies.

6. The use of an extract according to claims 1 or 2, for the preparation of antioxidant and radical scavenging agents for the treatment or prevention of free-radical mediated pathologies.

7. The use according to claim 6, for the preparation of cosmetics for preventing premature skin ageing and photooxidative degeneration of skin and hair.

8. The use according to claims 6 or 7, for the preparation of dietary supplements for preventing physical ageing, and for maintaining the physical performance.

9. Pharmaceutical, cosmetic or dietary/alimentary compositions containing the extract of claims 1 or 2, alone or in association with other natural extracts, hydrophilic and lipophilic antioxidants, ingredients or flavours, formulated with suitable vehicles excipients or additives.

10. Compositions according to claim 9, which are in liquid, liquid-viscous or solid form.

11. Compositions according to claim 10, selected from tablets, capsules, sachets, syrups, suppositories, vials and ointments, emulsions, gels, lotions or powders and dietary supplements, foods, beverages.

## Patentansprüche

1. Entkoffeinierter wässriger Extrakt aus *Ilex Paraguayen*sis-Pflanzen mit dem in Figur 1 angegebenen HPLC-Profil und mit einem Gehalt von nicht mehr als 0,1 Gew.-% Koffein.

2. Extrakt nach Anspruch 1, enthaltend 50 ± 0,8% (G/G) Polyphenole, bestimmt nach der Folin-Ciocalteau-Methode, 5,1 ± 0,2% (G/G) Chlorogensäure, bestimmt durch HPLC-Analyse, 1,96 ± 0,3% (G/G) Rutin.

3. Extrakt nach Anspruch 1 oder 2 zur Verwendung als Medikament.

4. Extrakt nach Anspruch 3 zur Prävention oder Behandlung von Erkrankungen im Zusammenhang mit oxidativer Schädigung.

5. Extrakt nach Anspruch 4 zur Prävention oder Behandlung von Atherosklerose, Krebs, kardiovaskulären Erkrankungen, rheumatoider Arthritis, Alzheimer, Parkinson und anderen altersbezogenen chronischen degenerativen Erkrankungen.

6. Verwendung eines Extraktes nach Anspruch 1 oder 2 zur Herstellung von Antioxidations- und radikalabfangenden Mitteln zur Behandlung oder Prävention von durch freie Radikale vermittelten Erkrankungen.

7. Verwendung nach Anspruch 6 zur Herstellung von Kosmetika zur Prävention von frühzeitiger Hautalterung und photooxidativer Degeneration von Haut und Haar.

8. Verwendung nach Anspruch 6 oder 7 zur Herstellung von Nahrungsergänzungsmitteln zur Prävention des physischen Alterns und zur Aufrechterhaltung der physischen Leistungsfähigkeit.

9. Pharmazeutische Zusammensetzungen, kosmetische Zusammensetzungen oder diätetische/alimentäre Zusammensetzungen, enthaltend den Extrakt nach Anspruch 1 oder 2 alleine oder gemeinsam mit anderen natürlichen Extrakten, hydrophilen und lipophilen Antioxidationsmitteln, Inhaltsstoffen oder Geschmacksstoffen bzw. Aromen, formuliert mit geeigneten Vehikeln, Exzipienzien oder Zusatzstoffen.

10. Zusammensetzungen nach Anspruch 9, die in flüssiger, flüssig-viskoser oder fester Form vorliegen.

11. Zusammensetzungen nach Anspruch 10, ausgewählt aus Tabletten, Kapseln, Briefchen, Sirupen, Suppositorien, Ampullen und Salben, Emulsionen, Gelen, Lotionen oder Pulvern und Nahrungsergänzungsmitteln, Lebensmitteln, Getränken.

## Revendications

1. Extrait aqueux décaféiné provenant de plantes de *Ilex Paraguayensis* ayant le profil de HPLC indiqué sur la figure 1 et ne contenant pas plus de 0,1 % en poids de caféine.

2. Extrait selon la revendication 1, contenant 50 ± 0,8 % (p/p) de polyphénols, comme déterminé par la méthode de Folin-Ciocalteau, 5,1 ± 0,2 % (p/p) d'acide chlorogénique, comme déterminé par une analyse par HPLC, 1,96 ± 0,3 % (p/p) de rutine.

3. Extrait selon la revendication 1 ou 2, pour une utilisation en tant que médicaments.

4. Extrait selon la revendication 3, pour la prévention ou le traitement de pathologies associées au stress oxydatif.

5. Extrait selon la revendication 4, pour la prévention ou le traitement de l'athérosclérose, du cancer, des maladies cardiovasculaires, de la polyarthrite rhumatoïde, de la maladie d'Alzheimer, de la maladie de Parkinson et d'autres pathologies dégénératives chroniques liées à l'âge.

6. Utilisation d'un extrait selon la revendication 1 ou 2, pour la préparation d'un antioxydant et d'agents de piégeage de radicaux pour le traitement ou la prévention de pathologies dont l'origine est constituée par les radicaux libres.

7. Utilisation selon la revendication 6, pour la préparation de cosmétiques destinée à empêcher le vieillissement prématuré de la peau et la dégénération photooxydative de la peau et des cheveux.

8. Utilisation selon la revendication 6 ou 7, pour la préparation de compléments diététiques destinés à empêcher le vieillissement physique et à maintenir les performances physiques.

9. Compositions pharmaceutiques, cosmétiques ou diététiques/alimentaires contenant l'extrait selon la revendication 1 ou 2, seul ou en association avec d'autres extraits naturels, des antioxydants hydrophiles et lipophiles, des ingrédients ou des arômes, formulés avec des véhicules, excipients ou additifs appropriés.

10. Compositions selon la revendication 9, qui sont sous forme liquide, liquide-visqueuse ou solide.

11. Compositions selon la revendication 10, choisies parmi les comprimés, les capsules, les sachets, les sirops, les suppositoires, les ampoules et les pommades, les émulsions, les gels, les lotions ou les poudres et les compléments diététiques, les aliments et les boissons.
